# EUROPÄISCHE PATENTSCHRIFT

(11) **EP 1 967 229 B1**
(45) Veröffentlichungstag und Bekanntmachung des Hinweises auf die Patenterteilung: **03.11.2010**
(21) Anmeldenummer: 08101134.8
(22) Anmeldetag: 31.01.2008
(51) Int. Cl.: A61N 5/10

(54) **Phantom und Verfahren für die Qualitätsüberprüfung einer medizintechnischen Anlage**
Phantom and method for quality testing of a medical technical assembly
Fantôme et procédé de surveillance de la qualité d'une installation médicale

(30) Priorität: 07.03.2007 DE 102007011154
(43) Veröffentlichungstag der Anmeldung: 10.09.2008
(73) Patentinhaber: SIEMENS AKTIENGESELLSCHAFT, 80333 München (DE)
(72) Erfinder: Use, Tim, 90469, Nürnberg (DE); Gunzert-Marx, Konstanze, 91054, Erlangen (DE); Knop, Sophia, 91052, Erlangen (DE)

(56) Entgegenhaltungen:
- EP-A- 0 514 971
- WO-A-00/07037
- WO-A-2005/018735
- WO-A-2007/012147
- US-A- 5 107 839
- US-A1- 2005 259 793

## Beschreibung

Die Erfindung betrifft das Gebiet der Qualitätsüberprüfung einer medizintechnischen Anlage, insbesondere das Gebiet der Qualitätssicherung im Rahmen der Partikeltherapie. Im Speziellen betrifft die Erfindung ein Phantom und ein Verfahren hierfür.

Die Partikeltherapie wird insbesondere zur Bestrahlung von Tumorgewebe bei Menschen eingesetzt. Zur Bestrahlung können insbesondere auch Ionen eingesetzt werden, wie beispielsweise Protonen, Heliumionen, Pionen, Kohlenstoffionen oder auch andere Ionensorten. Die Bestrahlung mit Ionen kennzeichnet sich im Vergleich zu einer herkömmlichen Bestrahlung mit Photonen oder Elektronen dadurch aus, dass insbesondere die Eindringtiefe der Strahlung in das menschliche Gewebe genauer gesteuert werden kann. Hierdurch ist eine genauere Bestrahlung des Tumors möglich, bei der umliegendes, zu schonendes Gewebe besser vor Strahlung geschützt werden kann.

Die Ionen werden hierzu in einem Beschleunigersystem, das beispielsweise ein Synchrotron oder ein Zyklotron umfasst, auf hohe Energien beschleunigt, und in einem Strahl auf den zu behandelnden Körper gerichtet. Der Strahl wird dabei durch ein Magnetsystem abgelenkt und gesteuert. Wesentliche und für die Sicherheit relevante Parameter des Strahls sind im Wesentlichen abhängig von den Einstellungen an der Anlage selbst, die als Resultat die Strahleigenschaften bestimmen. Hierzu gehören unter anderem der Strahldurchmesser, die Strahlposition und die Energie des Strahls.

Die Eigenschaften des bereitgestellten Partikelstrahls müssen bekannt sein. Um dies zu gewährleisten, werden in regelmäßigen Abständen Qualitätssicherungsmaßnahmen (auch als QA-Maßnahmen bezeichnet, QA für engl. "quality assurance") durchgeführt.

Die Überprüfung der Strahleigenschaften wird unter anderem mit so genannten Phantomen durchgeführt. Derartige Phantome werden mit dem Partikelstrahl bestrahlt und weisen eine empfindliche Einheit auf, mit dem die Wechselwirkung des Partikelstrahls mit dem Phantom aufgezeichnet und überprüft werden kann. Die empfindlichen Einheiten sind üblicherweise Filme, die durch die Bestrahlung mit dem Partikelstrahl belichtet werden. Es können aber auch, je nach Art des Phantoms, Detektoren eines anderen Typs, wie beispielsweise Ionisationskammern oder Fingerhutkammern, eingesetzt werden.

Bisher werden zur Überwachung verschiedener Strahlparameter in unterschiedlichen Intervallen jeweils unterschiedliche Phantome eingesetzt. Hierdurch entstehen mehrere Nachteile. Für jede QA-Prozedur muss das verwendete Phantom in eine definierte Lage zum Strahl gebracht werden, so dass für unterschiedliche Phantome jeweils eine Positionierung durchgeführt werden muss und somit mehrmals ein manuelles Intervenieren erforderlich ist. Zudem muss bei Phantomen, bei denen als empfindliche Einheit ein Film, z.B. ein Röntgenfilm, verwendet wird, für jede QA-Prozedur und für jedes Phantom je ein Film verwendet, entwickelt und ausgewertet werden.

WO-00/07037 offenbart ein Phantom, dass alle Merkmale in den Oberbegriffe des Anspruchen 1 und 8 umfasst.

Es ist die Aufgabe der Erfindung ein Phantom bereitzustellen, mit dem Qualitätssicherungsmaßnahmen auf einfache, kostengünstige und Zeit sparende Weise durchgeführt werden können. Weiterhin ist die Aufgabe der Erfindung ein Verfahren zur Qualitätskontrolle insbesondere für die Partikeltherapie anzugeben, das auf einfache, kostengünstige und Zeit sparende Weise durchgeführt werden kann.

Die Aufgabe wird erfindungsgemäß durch ein Phantom nach Anspruch 1 und durch ein Verfahren nach Anspruch 8 gelöst. Vorteilhafte Ausgestaltungen finden sich in den Merkmalen der abhängigen Ansprüche.

Das erfindungsgemäße Phantom für die Qualitätsüberprüfung einer medizintechnischen Anlage, insbesondere einer Therapieanlage, höchst insbesondere einer Partikeltherapieanlage, weist eine Fixiervorrichtung zur Ankoppelung des Phantoms an eine Positioniervorrichtung auf. Mithilfe der Fixiervorrichtung und der Ankoppelung des Phantoms an eine Positioniervorrichtung wird eine Positionierung des Phantoms im Raum erleichtert. Durch die Fixiervorrichtung kann das Phantom in eine definierte Lage relativ zu Positioniervorrichtung gebracht werden. Im Gegensatz zu einer rein manuellen Positionierung des Phantoms im Raum kann der Schritt der Positionierung nun einfacher, genauer, reproduzierbarer und wiederholbarer durchgeführt werden.

In einer vorteilhaften Variante ist die Fixiervorrichtung zur Ankoppelung des Phantoms an eine bewegliche Positioniervorrichtung ausgebildet. Hierdurch kann eine Repositionierung des Phantoms im Raum über die Positioniervorrichtung ausgeführt werden. Vorteilhaft ist die Positioniervorrichtung als Roboterarm ausgebildet, insbesondere als automatisch ansteuerbarer Roboterarm. Die Sollposition oder die Sollpositionen des Phantoms im Raum können beispielsweise in dem Steuersystem des Roboterarms hinterlegt werden, so dass eine Positionierung des Phantoms automatisch präzise und reproduzierbar durchgeführt werden kann.

Je nach eingesetztem Phantom kann eine bestimmte Eigenschaft des Partikelstrahls überprüft werden, wie z.B. die Energie des Partikelstrahls, die Ausdehnung des Partikelstrahls oder die Position des Partikelstrahls. Die Auswertung der gemessenen Strahlparameter kann dabei je nach Ausbildung des Phantoms online, d.h. während der Durchführung der zugehörigen QA-Maßnahme, durchgeführt werden oder aber auch nach erfolgter Durchführung der zugehörigen QA-Maßnahme.

Vorteilhafterweise ist die Positioniervorrichtung eine Patientenpositioniervorrichtung. Eine Patientenpositioniervorrichtung ist üblicherweise in Behandlungsräumen einer Partikeltherapieanlage bereits vorhanden, so dass diese Patientenpositioniervorrichtung für die Positionierung des Phantoms verwendet werden kann ohne die Notwendigkeit einer zusätzlichen Positioniervorrichtung.

Das Phantom weist eine Halterung zur Aufnahme zumindest einer Phantomeinheit auf. Mit Vorteil ist die Halterung zur auswechselbaren Aufnahme zumindest einer Phantomeinheit ausgebildet. Hierdurch kann das Phantom flexibel eingesetzt werden, da unterschiedliche Phantomeinheiten zur Überprüfung unterschiedlicher Strahlqualitäten und/oder Strahlparameter in der Halterung, beispielsweise einem Halterungsrahmen, befestigt werden können. Eine Fixierung des Phantoms an die Positioniervorrichtung erfolgt über die Halterung.

Die Halterung ist derart ausgebildet, dass mehrere Phantomeinheiten aufgenommen werden können. Hierdurch kann dasselbe Phantom für unterschiedliche QA-Maßnahmen eingesetzt werden. Mit jeder Phantomeinheit kann eine spezifische QA-Maßnahme durchgeführt werden. Hierdurch können mit dem einen Phantom insgesamt verschiedene QA-Maßnahmen durchgeführt werden.

Vorteilhafterweise weist das Phantom eine weitere Halterung zur Aufnahme einer Filmeinheit auf. Mit Vorteil ist ein Teil des Phantoms, insbesondere ein Teil der Halterung, als so genanntes "Wedge-Phantom" ausgebildet. Mit Vorteil ist ein Teil des Phantoms, insbesondere ein Teil der Halterung, zur Aufnahme einer Ionisationkammer ausgebildet.

In einer vorteilhaften Weiterbildung weist das Phantom, insbesondere die Halterung des Phantoms, zumindest eine Positionsmarkierung auf. Mithilfe einer derartigen Positionsmarkierung kann eine Positionierung des Phantoms im Raum auf einfache Weise überprüft werden, beispielsweise indem die gewünschte Position des Phantoms im Raum mithilfe von Laserfächern auf das Phantom projiziert wird. Das Muster der Laserfächer auf dem Phantom kann dann mit der Positionsmarkierung z.B. visuell und/oder automatisch verglichen werden.

Bei dem erfindungsgemäßen Verfahren zur Qualitätsüberprüfung von Strahleigenschaften bei einer Partikeltherapieanlage wird ein Phantom über eine Fixiervorrichtung an eine Positioniervorrichtung angekoppelt und mithilfe der Positioniervorrichtung an eine vordefinierte Position gebracht. Über die Fixiervorrichtung wird das Phantom relativ zu Positioniervorrichtung in eine definierte Lage gebracht werden, so dass eine reproduzierbare und genaue Positionierung des Phantoms im Raum erreicht werden kann. Mit Vorteil ist die Positioniervorrichtung ansteuerbar ausgebildet, wodurch die Positionierung an einer vordefinierten Position automatisch durchgeführt werden kann. Insbesondere ist die Positioniervorrichtung als Roboterarm ausgebildet.

In einer vorteilhaften Ausführungsform ist bei dem Verfahren die Positioniervorrichtung eine Patientenpositioniervorrichtung.

Das Phantom weist eine Halterung zur Aufnahme mehrerer Phantomeinheiten auf und wird mithilfe der Positioniervorrichtung sukzessive an verschiedenen vordefinierten Positionen positioniert. Die vordefinierten Positionen sind jeweils mit der Position der Phantomeinheiten in der Halterung abgestimmt, so dass an einer der vordefinierten Positionen jeweils eine Qualitätsüberprüfung bestimmter Strahlparameter, d.h. der Konstanz der Strahlparameter, durchgeführt wird. Welche Strahlparameter dies im Einzelnen sind, hängt mit den jeweiligen Phantomeinheit zusammen, mit der die Qualitätsüberprüfungsmaßnahme durchgeführt wird.

In einer vorteilhaften Ausführungsvariante weist das Phantom zumindest eine Positionsmarkierung auf. In diesem Fall wird bei dem Verfahren die korrekte Positionierung des Phantoms an der vordefinierten Position oder an den vordefinierten Positionen mithilfe einer Positionsmarkierung überprüft. Beispielsweise kann ein im Raum installiertes System von Laserfächer räumliche Koordinaten markieren, die eine korrekte Position des Phantoms anzeigen. Die Übereinstimmung der korrekten Position des Phantoms mit der tatsächlichen Position des Phantoms kann beispielsweise durch einen visuellen oder automatischen Abgleich des Musters der Laserfächer mit der Positionsmarkierung überprüft werden.

Eine Partikeltherapieanlage kann einen Behandlungsraum aufweisen, der eine Positioniervorrichtung umfasst, an die ein Phantom für die Qualitätsüberprüfung ankoppelbar ist, das über die Positioniervorrichtung an eine vordefinierte Position gebracht werden kann.

Ausführungsformen der vorliegenden Erfindung und Weiterbildungen gemäß den Merkmalen der abhängigen Ansprüche werden nun anhand der folgenden Zeichnung näher erläutert, ohne jedoch darauf beschränkt zu sein. Es zeigen:
- Figur 1: einen schematischen Überblick über den Aufbau einer Partikeltherapieanlage,
- Figur 2: eine perspektivische Ansicht eines Phantoms,
- Figur 3: eine perspektivische Ansicht eines weiteren Phantoms,
- Figur 4: eine vordere Ansicht eines weiteren Phantoms,
- Figur 5: die Positionierung des Phantoms anhand einer Patientenpositioniervorrichtung in einem Behandlungsraum, und
- Figur 6: eine Übersicht über Verfahrensschritte, mit denen die Durchführung einer QA-Prozedur vereinfacht und automatisiert werden kann.

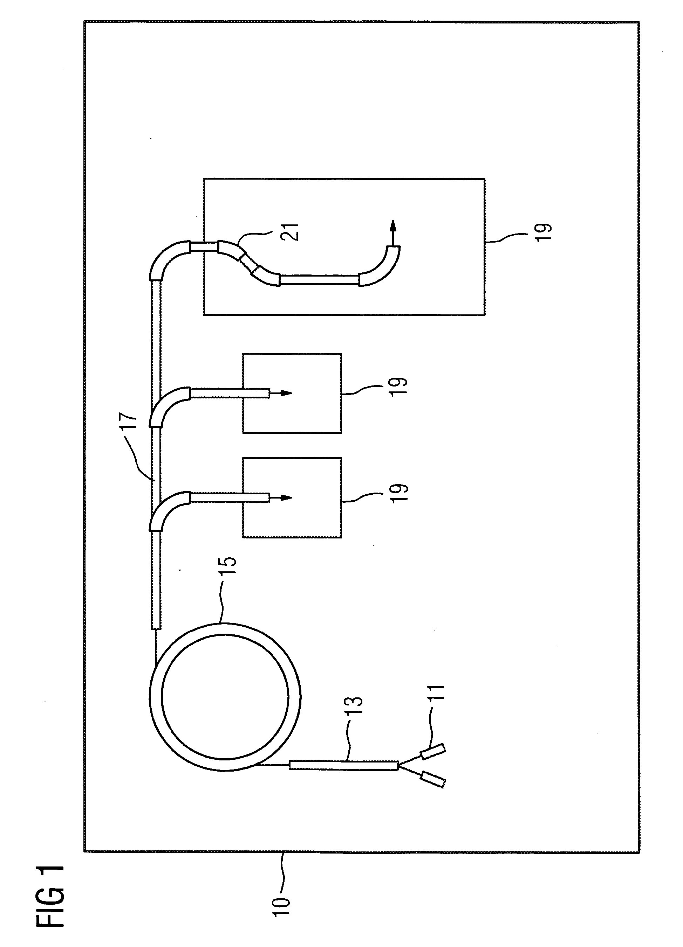

Figur 1 zeigt einen schematischen Überblick über den Aufbau einer Partikeltherapieanlage 10. In einer Partikeltherapieanlage 10 erfolgt die Bestrahlung insbesondere von tumorerkranktem Gewebe mit einem Partikelstrahl. Als Partikel werden vornehmlich Ionen wie beispielsweise Protonen, Pionen, Heliumionen, Kohlenstoffionen oder andere Ionensorten eingesetzt.

Üblicherweise werden derartige Partikel in einer Partikelquelle 11 erzeugt und in einem Vorbeschleuniger 13, z.B. einem linearen Beschleuniger (LINAC für engl.:"LINear ACcelerator"), beschleunigt. Anschließend werden die Partikel in einen Beschleuniger 15, beispielsweise ein Synchrotron oder Zyklotron, eingespeist, in dem sie auf hohe Energien, wie sie zur Bestrahlung nötig sind, beschleunigt werden. Nachdem die Partikel den Beschleuniger 15 verlassen, führt ein Hochenergiestrahl-Transportsystem 17 den Partikelstrahl zu den gewünschten Bestrahlungsräumen 19. In einem Bestrahlungsraum 19 werden die beschleunigten Partikel auf einen Körper gerichtet. Je nach Ausgestaltung erfolgt dies von einer festen Richtung (in so genannten "fixed beam"-Räumen) aus oder aber über eine bewegliche rotierbare Gantry 21 von verschiedenen Richtungen aus.

Dieser Grundaufbau einer Partikeltherapieanlage 10 ist typisch für viele Partikeltherapieanlagen, kann aber auch hiervon abweichen.

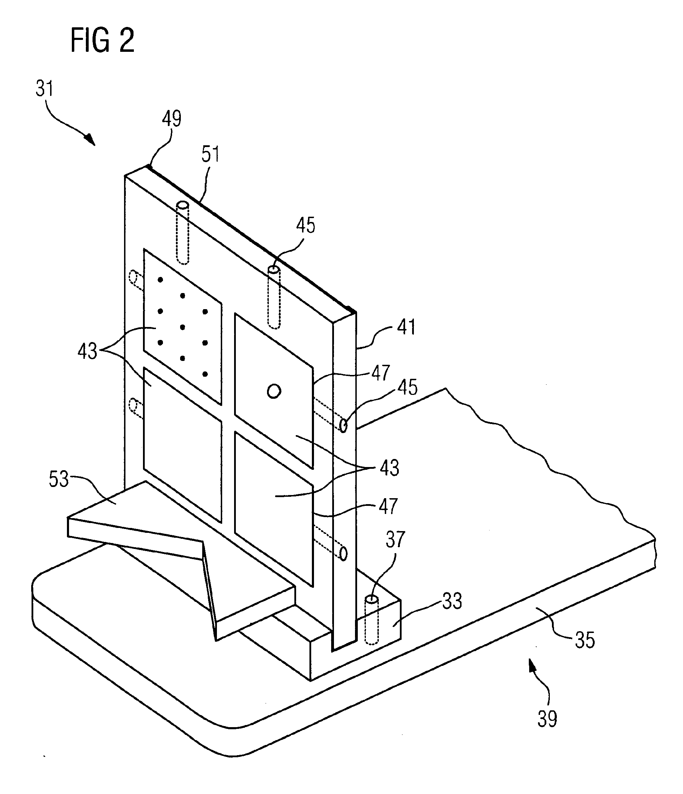

Figur 2 zeigt eine perspektivische Ansicht eines Phantoms 31, dass im Rahmen von QA-Maßnahmen bei einer Partikeltherapieanlage 10 eingesetzt werden kann. Im Bodenbereich des Phantoms 31 ist eine Fixiervorrichtung 33 angeordnet, über die das Phantom 31 beispielsweise an die Tischplatte 35 einer Patientenpositioniervorrichtung 39 beispielsweise über Schrauben 37 fixiert werden kann. Die hier gezeigte Ausgestaltung der Fixiervorrichtung 33 ist jedoch nur eine mögliche Ausgestaltung und kann in vielfältiger Weise variiert werden. Über die Patientenpositioniervorrichtung 39 kann das Phantom 31 automatisch an eine oder mehrere vordefinierte Position gefahren werden, die beispielsweise im Steuerungssystem der Partikeltherapieanlage 10 hinterlegt ist.

Das Phantom 31 weist weiterhin einen Halterungsrahmen 41 auf, in dem verschiedene Phantomeinheiten 43 austauschbar befestigt werden können. Bei dem hier gezeigten Phantom 31 können in dem Halterungsrahmen 41 beispielsweise vier verschiedene Phantomeinheiten 43 eingebracht und über Schrauben 45 befestigt werden.

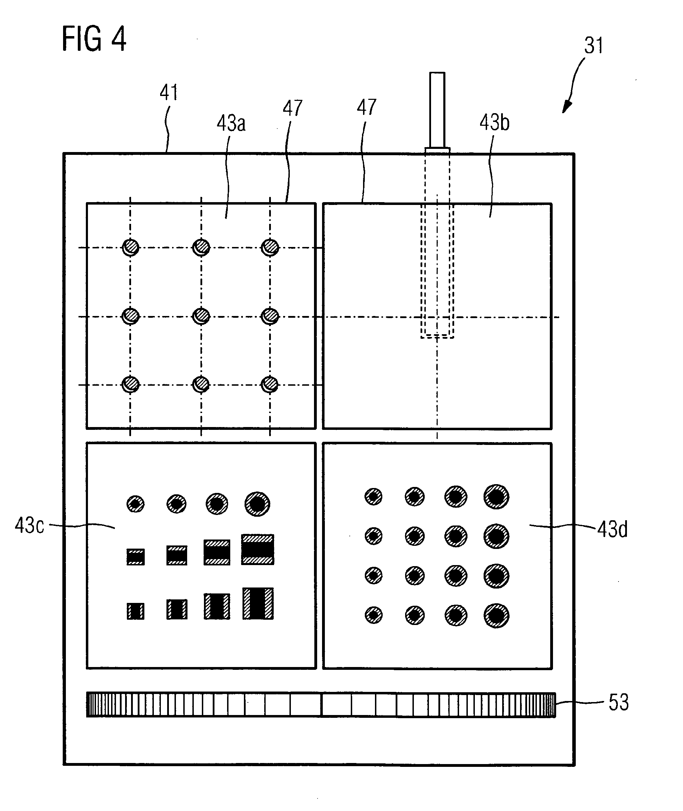

Die Aussparungen 47 im Halterungsrahmen 41, in die die Phantomeinheiten 43 eingebracht werden, sind dabei identisch, so dass die Phantomeinheiten 43 auf einfache Weise miteinander vertauscht und/oder ausgetauscht werden können. Verschiedene Ausgestaltungen der Phantomeinheiten 43 werden später anhand von Figur 4 näher erläutert. Das Phantom 31 weist zudem eine weitere Halterung 49 auf, in der ein Film 51 eingebracht werden kann. Hierdurch weist der Film 51 gegenüber dem Halterungsrahmen 41 eine definierte Position auf, wodurch sich die Auswertung des Filmes 51 vereinfacht.

Der untere Teil des Phantoms 31 ist als so genanntes Wedge-Phantom 53 ausgebildet. Auch hier ist es möglich, dass das Wedge-Phantom 53 austauschbar am Halterungsrahmen 41 befestigt werden kann.

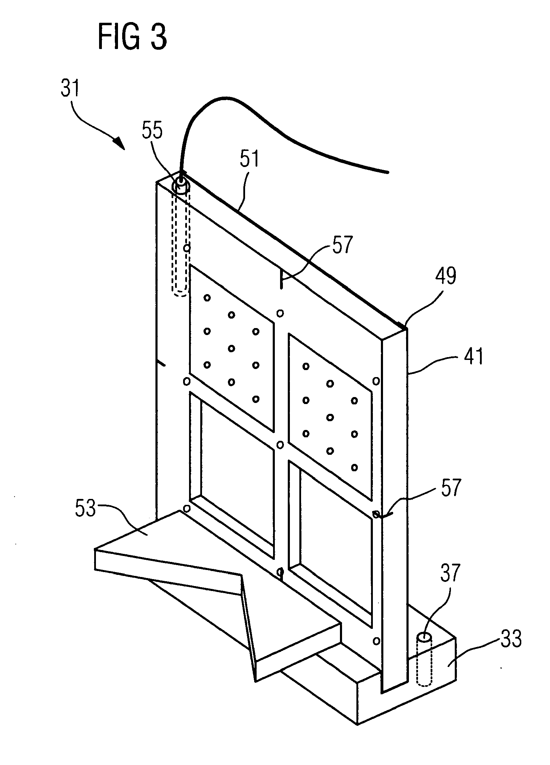

Figur 3 zeigt eine perspektivische Ansicht eines weiteren Phantoms 31, dass weitere vorteilhafte Ausgestaltungen aufweist. Im Halterungsrahmen 41 des Phantoms 31 ist eine Öffnung vorgesehen, in die eine Ionisationkammer 55, die beispielsweise als Fingerhutkammer ausgebildet ist, eingebracht werden kann. Die Öffnung kann beispielsweise außerhalb des Bereichs angebracht werden, der durch den Film 51 abgedeckt ist, so dass sich die Ionisationkammer 55 auf dem Film 51 nicht abbildet.

Weiterhin sind am Halterungsrahmen 41 des Phantoms 31 Positionsmarkierungen 57 angeordnet, mit denen eine Positionierung des Phantoms überprüft werden kann. Beispielsweise können Laserfächer bestimmte Koordinaten im Raum markieren, die die korrekte Position des Phantoms 31 im Raum kennzeichnen. Anhand einer visuellen Inspektion kann mithilfe der Positionsmarkierungen 57 überprüft werden, ob sich die Positionsmarkierungen 57 mit den Koordinaten, die durch die Laserfächer markiert sind, decken. Diese Überprüfung kann auch automatisiert mit ausgebildeten Fotodetektoren durchgeführt werden.

Figur 4 zeigt eine Ansicht von vorne eines weiteren Phantoms 31. Das Phantom 31 weist dabei eine rechteckige Fläche von etwa 265 mm mal 325 mm auf, die der Größe eines handelsüblichen Films entspricht. Der Halterungsrahmen 41 hat dabei vier Aussparungen 47, in die je eine Phantomeinheit 43a ... 43 d eingebracht ist. Die vier Phantomeinheiten 43 sind dabei unterschiedlich ausgebildet und dienen dazu, unterschiedliche Eigenschaften des Partikelstrahls hinsichtlich ihrer Qualität und/oder Konstanz zu überprüfen. Mit einer ersten Phantomeinheit 43a kann die Genauigkeit der Position des Partikelstrahls im Scanbereich verifiziert werden. Eine zweite Phantomeinheit 43b dient zur Überprüfung der Luft-Dichte-Korrektur (engl.: "air density correction") bei der Ionisationsdosimetrie. Eine dritte Phantomeinheit 43c dient zur Überprüfung der Feldgeometrie (engl.: "field geometry"). Eine vierte Phantomeinheit 43d dient zur Überprüfung der Fokusbreite des Partikelstrahls. Die grauschattierten Bereiche kennzeichnen bei der dritten und vierten Phantomeinheit 43c, 43d einen gaußförmigen Abfall der Schwärzung des Films. Im Bodenbereich ist ein keilförmig ausgebildetes Wedge-Phantom 53 angebracht, dessen keilförmige Ausbildung in der der Darstellung mit vertikalen Linien angedeutet ist. Der Übersichtlichkeit halber nicht gezeigt ist in Figur 4 die Fixiervorrichtung 33 des Phantoms 31.

Die einzelnen QA-Prozeduren für verschiedene Strahlparameter werden mit dem Phantom 31 sukzessive durchgeführt, indem das Phantom 31 mithilfe der Positioniervorrichtung 39 derart positioniert wird, dass sich jeweils eine der Phantomeinheiten 43a ... 43d im Scanbereich des Partikelstrahls befindet. Nach dem eine QA-Prozedur mit dieser Phantomeinheit abgeschlossen ist, wird das Phantom mithilfe der Positioniervorrichtung 39 repositioniert, um anschließend eine weitere QA-Prozedur mit einer weiteren der Phantomeinheiten 43a ... 43d durchzuführen. Das Repositionieren des Phantoms 31 erfolgt dabei bevorzugter Weise automatisch mithilfe der Positioniervorrichtung 39 und entsprechend hinterlegten Steuerungsbefehlen.

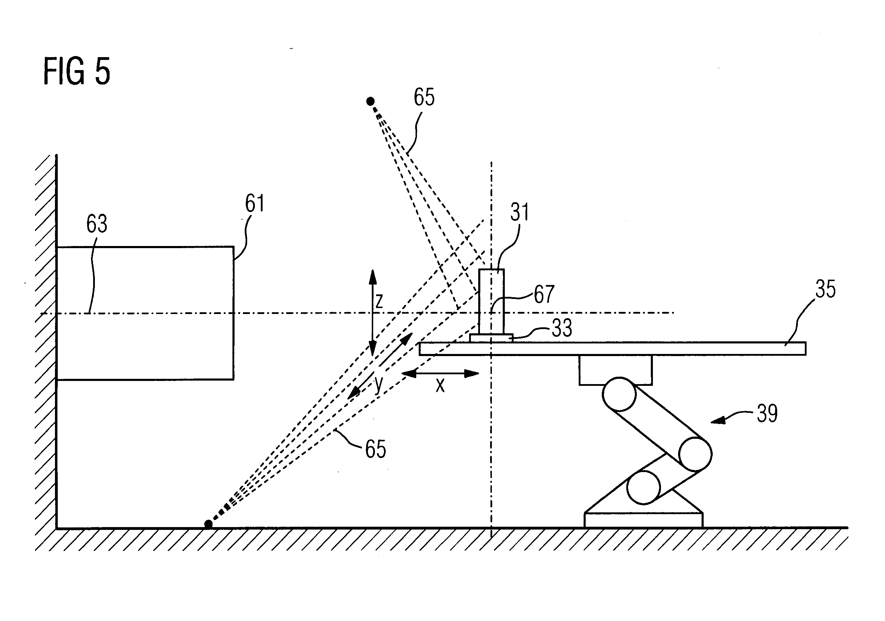

Figur 5 zeigt eine schematische Übersicht über einen Behandlungsraum mit einer Patientenpositioniervorrichtung 39, die als Roboterarm ausgebildet ist. Der Partikelstrahl tritt entlang der Strahlachse 63 über eine so genannte "Nozzle" aus der Strahlzuführungseinheit 61 aus.

Das Phantom 31 ist über die Fixiervorrichtung 33 z.B. an den Patiententisch 35 oder dessen Tischplatte angekoppelt und kann über die Patientenpositioniervorrichtung 39 räumlich positioniert werden. Laserfächer 65 können die Koordinaten anzeigen, an denen das Phantom positioniert werden soll. Anhand der Laserfächer 65 und der Positionsmarkierungen 57 an dem Phantom 31 kann beispielsweise die korrekte Positionierung visuell oder automatisch mit geeigneten Detektoren überprüft werden. Üblicherweise wird das Phantom 31 im Isozentrum 67 des Behandlungsraums positioniert.

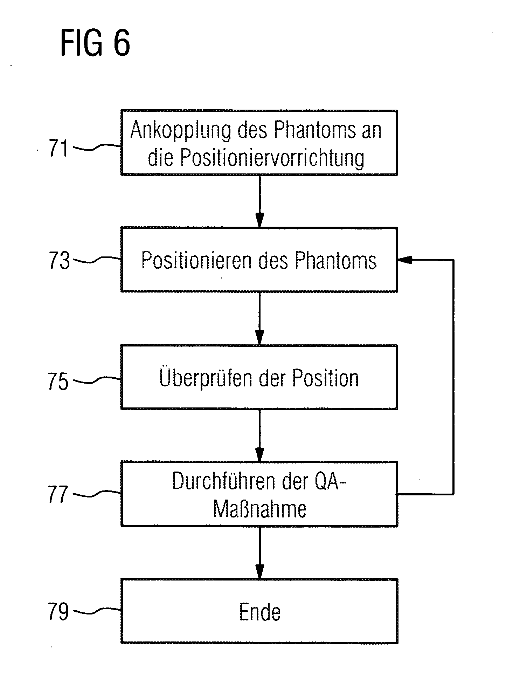

Figur 6 zeigt einen schematischen Ablauf eines QA-Verfahrens, das mit einem Phantom, wie es in Figur 2 bis Figur 4 gezeigt ist, durchgeführt wird.

In einem ersten Schritt 71 wird das Phantom 31 über die Fixiervorrichtung 33 an eine Positioniervorrichtung 39 angekoppelt, vorzugsweise an eine Patientenpositioniervorrichtung.

In einem zweiten Schritt 73 wird das Phantom 31 mithilfe der Positioniervorrichtung 39 derart im Behandlungsraum positioniert, dass sich eine der Phantomeinheiten 43 im Scanbereich des Partikelstrahls befindet.

In einem dritten Schritt 75 wird optional die Position des Phantoms 31 im Raum überprüft, beispielsweise mithilfe von Positionsmarkierungen 57 und Laserfächern 65. Eine derartige Überprüfung der Position des Phantoms 31 kann z.B. visuell oder aber auch automatisch erfolgen.

In einem vierten Schritt 77 wird eine auf die Phantomeinheit 43 abgestimmte QA-Maßnahme durchgeführt.

Der zweite bis vierte Schritt 73, 75, 77 werden solange wiederholt, bis alle gewünschten QA-Prozeduren durchgeführt worden sind (fünfter Schritt 79 - Ende).

Vorteile eines solchen Verfahrens bzw. eines solchen Phantoms 31 mit dem dazugehörigen Positionierungssystem 39 sind, dass mehrere QA-Prozeduren mit einem einzigen Phantom 31 und einem einzigen Film 51 durchgeführt werden können, die Positionierung der verschiedenen Phantomeinheiten 43 automatisch durchgeführt wird, so dass insgesamt Zeit, Material und manuelle Interventionen eingespart werden.

## Patentansprüche

1. Phantom für die Qualitätsüberprüfung einer medizintechnischen Anlage, insbesondere einer medizinischen Therapieanlage, höchst insbesondere einer Partikeltherapieanlage, aufweisend eine Fixiervorrichtung zur Ankoppelung des Phantoms an eine Positioniervorrichtung,
**dadurch gekennzeichnet,**
**dass** das Phantom eine Halterung aufweist, die zur Aufnahme mehrerer Phantomeinheiten, mit denen jeweils eine spezifische QA-Maßnahme (Qualitätssicherungsmaßnahme) durchführbar ist, ausgebildet ist.

2. Phantom nach Anspruch 1,
**dadurch gekennzeichnet,**
**dass** die Fixiervorrichtung zur Ankoppelung des Phantoms an eine beweglich ausgebildete Positioniervorrichtung ausgebildet ist, insbesondere an eine als Roboterarm ausgebildete Positioniervorrichtung.

3. Phantom nach Anspruch 1 oder 2,
**dadurch gekennzeichnet,**
**dass** die Fixiervorrichtung zur Ankoppelung des Phantoms an eine Patientenpositioniervorrichtung ausgebildet ist.

4. Phantom nach einem der Ansprüche 1 bis 3,
**dadurch gekennzeichnet,**
**dass** die Halterung zur auswechselbaren Aufnahme zumindest einer der mehreren Phantomeinheiten ausgebildet ist.

5. Phantom nach einem der Ansprüche 1 bis 4,
**dadurch gekennzeichnet,**
**dass** das Phantom eine weitere Halterung zur Aufnahme einer Filmeinheit aufweist.

6. Phantom nach einem der Ansprüche 1 bis 5,
**dadurch gekennzeichnet,**
**dass** ein Teil des Phantoms als wedge-Phantom ausgebildet ist und/oder ein weiterer Teil des Phantoms zur Aufnahme eine Ionisationkammer ausgebildet ist.

7. Phantom nach einem der Ansprüche 1 bis 6,
**dadurch gekennzeichnet,**
**dass** das Phantom zumindest eine Positionsmarkierung aufweist.

8. Verfahren zur Qualitätsüberprüfung einer medizintechnischen Anlage, insbesondere einer medizinischen Therapieanlage, höchst insbesondere einer Partikeltherapieanlage,
wobei ein Phantom über eine Fixiervorrichtung an eine Positioniervorrichtung angekoppelt wird und mithilfe der Positioniervorrichtung an eine vordefinierte Position gebracht wird, **dadurch gekennzeichnet,**
**dass** das Phantom eine Halterung aufweist zur Aufnahme mehrerer Phantomeinheiten, mit denen jeweils eine spezifische QA-Maßnahme (Qualitätssicherungsmaßnahme) durchgeführt werden kann,
und **dass** die Halterung mithilfe der Positioniervorrichtung sukzessive an verschiedenen vordefinierten Positionen positioniert wird, an denen jeweils eine der spezifischen QA-Maßnahmen zur Qualitätsüberprüfung bestimmter Strahlparameter durchgeführt wird.

9. Verfahren nach Anspruch 8,
**dadurch gekennzeichnet,**
**dass** die Positioniervorrichtung ansteuerbar und insbesondere als Roboterarm ausgebildet ist.

10. Verfahren nach Anspruch 8 oder 9,
**dadurch gekennzeichnet,**
**dass** die Positioniervorrichtung eine Patientenpositioniervorrichtung ist.

11. Verfahren nach einem der Ansprüche 8 bis 10,
**dadurch gekennzeichnet,**
**dass** das Phantom zumindest eine Positionsmarkierung aufweist und wobei die korrekte Positionierung des Phantoms an den vordefinierten Positionen mithilfe der Positionsmarkierung überprüft wird.

## Claims

1. Phantom for quality testing of a medical technical assembly, particularly of a medical treatment assembly, and most particularly of a particle treatment assembly, having a fixing apparatus for linking the phantom to a positioning apparatus,
**characterised in that**
the phantom has a holder which is designed to receive a plurality of phantom units with which a specific QA (quality assurance) measure can be performed in each case.

2. Phantom according to claim 1,
**characterised in that**
the fixing apparatus is designed for linking the phantom to a movably designed positioning apparatus, particularly to a positioning apparatus designed as a robot arm.

3. Phantom according to claim 1 or 2,
**characterised in that**
the fixing apparatus is designed for linking the phantom to a patient positioning apparatus.

4. Phantom according to one of claims 1 to 3,
**characterised in that**
the holder is designed for interchangeably receiving at least one of the plurality of phantom units.

5. Phantom according to one of claims 1 to 4,
**characterised in that**
the phantom has a further holder for receiving a film unit.

6. Phantom according to one of claims 1 to 5,
**characterised in that**
one part of the phantom is designed as a wedge phantom and/or a further part of the phantom is designed to receive an ionisation chamber.

7. Phantom according to one of claims 1 to 6,
**characterised in that**
the phantom has at least one position marking.

8. Method for quality testing of a medical technical assembly, particularly of a medical treatment assembly, and most particularly of a particle treatment assembly, wherein a phantom is linked to a positioning apparatus via a fixing apparatus and with the help of the positioning apparatus is brought to a predefined position,
**characterised in that**
the phantom has a holder which is designed to receive a plurality of phantom units with which a specific QA (quality assurance) measure can be performed in each case,
and that the holder is successively positioned at different predefined positions with the help of the positioning apparatus, at which positions in each case one of the specific QA measures is performed to test the quality of particular beam parameters.

9. Method according to claim 8,
**characterised in that** the positioning apparatus can be controlled and is particularly designed as a robot arm.

10. Method according to claim 8 or 9,
**characterised in that** the positioning apparatus is a patient positioning apparatus.

11. Method according to one of claims 8 to 10,
**characterised in that** the phantom has at least one position marking and wherein the correct positioning of the phantom at the predefined positions is tested with the help of the position marking.

## Revendications

1. Fantôme pour le contrôle de la qualité d'une installation médico-technique, en particulier d'une installation médico-thérapeutique, de manière tout à fait particulière d'une installation de thérapie particulaire, présentant un dispositif de fixation pour le couplage du fantôme à un dispositif de positionnement,
**caractérisé en ce que** le fantôme présente un support qui est réalisé pour la réception de plusieurs fantômes unitaires avec lesquels on peut procéder respectivement à une mesure QA spécifique (mesure d'assurance de la qualité).

2. Fantôme selon la revendication 1,
**caractérisé en ce que** le dispositif de fixation est réalisé pour le couplage du fantôme à un dispositif de positionnement réalisé pour être mobile, en particulier à un dispositif de positionnement réalisé sous la forme d'un bras robotique.

3. Fantôme selon la revendication 1 ou 2,
**caractérisé en ce que** le dispositif de fixation est réalisé pour le couplage du fantôme à un dispositif de positionnement de patient.

4. Fantôme selon l'une quelconque des revendications 1 à 3,
**caractérisé en ce que** le support est réalisé pour la réception échangeable d'au moins un desdits plusieurs fantômes unitaires.

5. Fantôme selon l'une quelconque des revendications 1 à 4,
**caractérisé en ce que** le fantôme présente un support supplémentaire pour la réception d'un film photographique unitaire.

6. Fantôme selon l'une quelconque des revendications 1 à 5,
**caractérisé en ce qu'**une partie du fantôme est réalisée sous la forme d'un fantôme cunéiforme et/ou **en ce qu'**une autre partie du fantôme est réalisée pour la réception d'une chambre d'ionisation.

7. Fantôme selon l'une quelconque des revendications 1 à 6,
**caractérisé en ce que** le fantôme présente au moins un marqueur de position.

8. Procédé pour le contrôle de la qualité d'une installation médico-technique en particulier d'une installation médico-thérapeutique, de manière tout à fait particulière d'une installation de thérapie particulaire,
dans lequel on couple un fantôme via un dispositif de fixation à un dispositif de positionnement et on l'amène, à l'aide du dispositif de positionnement, dans une position prédéfinie, **caractérisé en ce que** le fantôme présente un support qui est réalisé pour la réception de plusieurs fantômes unitaires avec lesquels on peut procéder respectivement à une mesure QA spécifique (mesure d'assurance de la qualité), et
**en ce que** le support vient se mettre, au moyen du dispositif de positionnement, successivement à différentes positions prédéfinies auxquelles on met en oeuvre respectivement une des mesures QA spécifiques, pour le contrôle de la qualité de paramètres d'irradiation déterminés.

9. Procédé selon la revendication 8, **caractérisé en ce que** le dispositif de positionnement peut être commandé et est réalisé en particulier sous la forme d'un bras robotique.

10. Procédé selon la revendication 8 ou 9, **caractérisé en ce que** le dispositif de positionnement est un dispositif de positionnement de patient.

11. Procédé selon l'une quelconque des revendications 8 à 10, **caractérisé en ce que** le fantôme présente au moins un marqueur de position, le positionnement correct du fantôme aux positions prédéfinies étant contrôlé à l'aide du marqueur de position.
